# EUROPÄISCHE PATENTSCHRIFT

(11) **EP 2 543 343 B1**
(45) Veröffentlichungstag und Bekanntmachung des Hinweises auf die Patenterteilung: **11.01.2017**
(21) Anmeldenummer: 12175102.8
(22) Anmeldetag: 05.07.2012
(51) Int. Cl.: A61F 2/30, A61F 2/44

(54) **Gelenkimplantat**
Joint implant
Implant pour articulation

(30) Priorität: 08.07.2011 DE 202011103010 U
(43) Veröffentlichungstag der Anmeldung: 09.01.2013
(73) Patentinhaber: Taurus GmbH & Co. KG, 63755 Alzenau (DE)
(72) Erfinder: Siedler, Uwe, 63775 Alzenau (DE); Bleistein, Frank, 63755 Alzenau (DE)
(74) Vertreter: Lippert, Stachow & Partner

(56) Entgegenhaltungen:
- WO-A1-2005/094732
- WO-A2-2007/092144
- DE-U1- 20 307 876
- DE-U1-202008 012 749
- US-A1- 2006 142 862
- US-B1- 6 966 818

## Beschreibung

Die Erfindung betrifft ein Gelenkimplantat nach dem Oberbegriff von Anspruch 1.

Derartige Gelenkimplantate werden beispielsweise als Bandscheibenprothesen eingesetzt, um benachbarte Wirbel einer Wirbelsäule gelenkig miteinander zu verbinden. Das Implantat kann hierbei ein oder mehrere Gelenkbereiche aufweisen, die unabhängig voneinander eine gelenkige Bewegung der jeweils angrenzenden Bereiche des Implantats zueinander ermöglichen, beispielsweise Verschwenk- oder Verkippbewegungen gegeneinander. Das Implantat kann somit insbesondere zwei unterschiedliche Gelenkbereiche aufweisen, welche Verschwenk- oder Verkippbewegungen um jeweils eine Verschwenk- oder Verkippachse durchführen, welche in einer quer zur Implantatlängsachse liegenden Ebene angeordnet ist, wobei die beiden Achsen in einem Winkel zueinander angeordnet sind.

Um das Implantat an die jeweiligen anatomischen Verhältnisse anzupassen und gleichzeitig eine einfache Handhabung des Implantats zur Vorbereitung desselben zur Implantation wie auch einfachen Handhabung des Implantats während der Implantation zu ermöglichen. Hierbei hat es sich als vorteilhaft herausgestellt, wenn das obere und/oder untere Implantatteil einerseits ein Gelenkteil aufweist, wobei das Gelenkteil den Gelenkbereich des jeweiligen Implantatteils bereitstellt, so dass die beiden aneinander liegenden Gelenkbereiche der beiden Implantatteile oder aber der jeweilige Gelenkbereich des Implantatteils mit der Anlagefläche des Zwischenelementes eine gelenkige Verbindung ausbildet. Hierdurch kann das Implantat an unterschiedlich große Knochen mit unterschiedlich großen Implantatanlageflächen und/oder an unterschiedliche Abstände der Verbindungsbereiche der Knochen zueinander einfach konfiguriert werden, nämlich durch Auswahl geeigneter Gelenkteile. Das Gelenk ist hierbei auf einfache Weise an Patienten mit unterschiedlich großen Wirbeln und/oder Patienten unterschiedlichen Gewichtes anpassbar.

Weiterhin ist es erforderlich, die verschiedenen Implantatteile aneinander festzulegen, zum einen, um das Implantat als vormontierte Baugruppe implantieren zu können, was die Implantation wesentlich vereinfacht. Zum anderen muss die Verbindung zwischen den einzelnen Implantatteilen hohen mechanischen Beanspruchungen über einen möglichst langen Zeitraum standhalten, da zum Einen auf das Implantat bei einer Bewegung des jeweiligen Patienten hohe Kräfte wirken, zum Anderen aufgrund der komplexen Bewegungen des Patienten auch die Implantatteile zueinander komplex zusammengesetzte Bewegungen durchführen, beispielsweise Verschwenkbewegungen, Torsionsbewegungen, zusammengesetzte Verschwenk-Torsionsbewegungen oder dergleichen. Die Verbindung der Implantatteile muss auch bei diesen komplexen Bewegungen unter Berücksichtigung hoher Kräfte eine lange Lebensdauer aufweisen.

Weiterhin muss ein derartiges Implantat bzw. die einzelnen Bauteile desselben einfach sterilisierbar sein.

Ein gattungsgemäßes Implantat ist bereits aus der DE 20 2008 012 749 bekannt, bei welchem das obere und das untere Implantatteil jeweils aus einem Gelenkteil und einem an dem jeweiligen Knochen anlegbaren Anlageteil besteht, welche durch ein Verbindungselement miteinander zu einer zusammenhängenden Baugruppe verbunden werden. Das Gelenkteil wird hierzu in eine seitlich offene Ausnehmung des Anlageteils geschoben, wobei die Einschubrichtung in einer Ebene parallel zu den beiden Hauptebenen der beiden genannten Bauteile liegt, wobei das Gelenkteil dann mittels einer Rastverbindung an dem Anlageteil verschiebungssicher festgelegt wird. Ein derartiges Implantat hat sich bereits verschiedentlich bewährt, nachteilig ist jedoch, dass sich bei komplexen Bewegungen des Patienten beim implantierten Implantat- und Anlageteil sich voneinander lösen können, was die Gefahr von Verletzungen, insbesondere Rückenmarkverletzungen, mit sich bringt. Eine Lösung dieses Problems dahingehend, die Rastverbindung zwischen Gelenk- und Anlageteil robuster auszuführen, hat sich jedoch als nicht praktikabel erwiesen, da dies auch die Montage des Implantats erschwert. Der Erfindung liegt daher die Aufgabe zugrunde, ein gattungsgemäßes Implantat zu schaffen, welches auch unter Einwirkung hoher Kräfte und komplexer Bewegungsprofile eine lange Lebensdauer aufweist und welches einfach montierbar ist. Weiterhin ist es Aufgabe der vorliegenden Erfindung eine Vorrichtung zu schaffen, mittels welcher die Montage des Implantats vereinfacht wird.

Diese Aufgabe wird durch ein Implantat nach Anspruch 1 gelöst. Erfindungsgemäß weisen das Gelenkteil und das Anlageteil zumindest eines oder beider der Implantatteile Befestigungsmittel auf, welche als Formschlussmittel ausgebildet sind, die unter Verdrehung von Gelenkteil und Anlageteil miteinander zusammenwirken bzw. zur Zusammenwirkung gebracht werden können. Dadurch, dass die Formschlussmittel des jeweiligen Gelenkteils und Anlageteils unter Verdrehung aneinander befestigbar sind, ist unter den Anwendungsbedingungen des implantierten Implantats dieses auch unter komplexen Bewegungen des Patienten gegen ein Lösen von Gelenkteil und Anlageteil weitestgehend gesichert. Insbesondere ist eine Trennung von Gelenkteil und Anlageteil in einer Richtung, welche senkrecht zur Längsachse des Implantats bzw. zur Wirbelsäulenlängsachse steht, praktisch verhindert. Die Aufnahmen des jeweils einen Teils von Gelenk- und Anlageteil für das jeweils andere der beiden Teile (beispielsweise eine in dem Anlageteil angeordnete Aufnahme zum Aufnehmen des Befestigungsbereiches des Gelenkteils) kann somit zumindest im Wesentlichen vollständig oder vollständig bezogen auf einen Umfang geschlossen sein, wobei der Umfang in der Hauptebene des Implantatteils liegt, also senkrecht zu der Implantatlängsachse oder Wirbelsäulenlängsachse angeordnet ist. Eine Beabstandung von Gelenkteil und Anlageteil zueinander in einer beliebigen Richtung in der Hauptebene des Implantatteils ist hierdurch ausgeschlossen, wobei sich durch umfangreiche Versuche im Rahmen der Erfindung gezeigt hat, dass durch diese Ausbildung des Implantats ein Lösen von Gelenk- und Anlageteil auch bei hohen und komplexen Belastungen (komplex im Bezug auf die Bewegungsarten und Bewegungsrichtungen der Lageveränderung der Teile des Implantats zueinander) wirksam verhindert werden kann. Durch die Formschlussmittel, welche unter Verdrehung von Gelenk- und Anlageteil gegeneinander zusammenwirken, ist zudem eine Verdrehsicherung der beiden genannten Teile gegeneinander gegeben, welche einer Lösung der beiden Teile voneinander entgegen wirkt. Die Formschlussmittel wirken somit gleichzeitig als Verdrehsicherung von Gelenk- und Anlageteil gegeneinander. Da andererseits die beiden Implantatteile gegeneinander verdrehbar sind, insbesondere die beiden Implantatteile gegen das Zwischenelement verdrehbar sind - vorzugsweise unabhängig voneinander gegen das Zwischenelement verdrehbar sind - ist der einer Verdrehung der Implantatteile gegeneinander entgegen gesetzte Widerstand (insbesondere Reibungswiderstand) bei der Benutzung des am Patienten implantierten Implantats geringer als der durch das Formschlussmittel entgegen gesetzte Verdrehwiderstand von Gelenk- und Anlageteil gegeneinander. Hierbei ist das Implantat derart ausgebildet, dass eine Verdrehung der beiden Implantatteile gegeneinander frei über einen Winkel ermöglicht ist, welcher der anatomischen maximalen Verdrehstellung zweier benachbarter Wirbel einer Wirbelsäule entspricht, insbesondere einer Wirbelsäule eines Säugetieres, im speziellen eines Menschen. Im speziellen ist das Implantat derart ausgebildet, dass eine Verdrehung des oberen und unteren Implantatteils gegenüber dem Zwischenelement (bezogen auf den Gesamtverdrehwinkel der beiden Implantatteile gegenüber dem Zwischenelement) gleich oder größer ist als der anatomische Verdrehwinkel benachbarter Wirbel einer Wirbelsäule eines Säugetieres, insbesondere eines Menschen. Besonders bevorzugt ist eine freie Verdrehung des oberen und/oder unteren Implantatteils gegeneinander in einem Winkel von ≥ 20 - 40° oder vorzugsweise ≥ 60 - 120° oder ≥ 180 - 240° oder besonders bevorzugt um zumindest ca. 360° möglich. Ferner ist, vorzugsweise in Kombination hiermit, eine freie Verdrehung des oberen und/oder unteren Implantatteils gegen das Zwischenelement in einem Winkel von ≥ 60 - 120° oder ≥ 180 - 240° oder besonders bevorzugt um zumindest ca. 360° möglich. Hierdurch kann einerseits das Implantat bei unterschiedlichen Wirbel oder Gelenken möglichst flexibel eingesetzt werden, zum anderen ist sichergestellt, dass bei der gegebenen freien Verdrehbarkeit der Implantatteile gegeneinander bzw. gegen das Zwischenelement der Winkelbereich der freien Verdrehbarkeit so groß ist, dass die Formschlussmittel zwischen Gelenk- und Anlageteil nicht belastet werden, zumindest nicht mit einer Kraft belastet werden, welche wesentlich größer ist als die Kraft, welche bei der freien Verdrehbarkeit des oberen und unteren Implantatteils zueinander zu überwinden ist (ggf. bei Anordnung eines Zwischenelements zwischen diesen). Gegebenenfalls ist die Kraft zur Überwindung des Formschlusses zwischen Gelenk- und Anlageteil ≥ dem 2- bis 5fachen oder ≥ dem 10- bis 20fachen oder besonders bevorzugt ≥ dem 50- bis 100fachen der Kraft der freien Verdrehbarkeit des oberen und/oder unteren Implantatteils zueinander. Besonders bevorzugt sind die Formschlussmittel der Verdrehung des Gelenk- und Anlageteils zueinander nur unter Zerstörung der Formschlussmittel überwindbar, falls diese nicht durch eine gesonderte Betätigung außer Eingriff gebracht werden.

Besonders bevorzugt weist das obere und/oder untere Gelenkteil einen Rücken mit einer Rückenlinie, vorzugsweise einer geradlinigen Rückenlinie, auf, wobei das Gelenkteil mit dem Rücken an der Anlagefläche des Zwischenelementes anliegenden abwälzen kann. Der Rücken kann hierzu im Querschnitt gewölbt, vorzugsweise kreisbogenförmig gewölbt, ausgebildet sein. Seitlich an den Rücken, einseitig oder vorzugsweise beidseitig, können Anlageflächen vorgesehen sein, welche flächig an die Oberflächen des Zwischenelementes anlegbar sind, unter Begrenzung der Abwälzbewegung des Gelenkteils gegenüber dem Zwischenelement. Die Anlagefläche des Zwischenelementes für den Gelenkteilrücken kann eben ausgeführt sein, was vorzugsweise für beide Anlagebereiche des Zwischenelementes an die Rücken der beiden Gelenkteile gilt, vorzugsweise mit planparalleler Ausrichtung der beiden Anlageflächen. Besonders bevorzugt ist das Zwischenelement als Scheibe ausgebildet, insbesondere als Scheibe mit planparallelen Oberflächen, welche den jeweiligen Gelenkteilen zugewandt sind.

Insgesamt wird hierdurch ein Implantat bereitgestellt, bei welchem auch bei komplexen Bewegungen der Gelenkteile zueinander das Implantat auch bei hohen einwirkenden Kräften über eine sehr lange Lebensdauer sehr sicher montiert ist und welches aufgrund großer Aufnahmen leicht sterilisierbar ist.

Vorzugsweise sind die beiden Implantatteile gegeneinander in einer Richtung quer, vorzugsweise senkrecht zur Implantatlängsachse gegeneinander verschiebbar. Hierzu kann der Gelenkbereich zumindest eines oder vorzugsweise beider Implantatteile mit einem Langloch zur Durchführung des Verbindungsmittels, welches das obere und untere Implantatteil unter Ausbildung einer zusammenhängenden Baugruppe miteinander verbindet, versehen sein. Das Verbindungsmittel ist dann in dem Langloch quer zur Implantatlängsachse verschiebbar. Die Längsseitenwände des Langloches können parallel zueinander und geradlinig verlaufen, so dass das Langloch eine konstante Weite hat. Vorzugsweise erstreckt sich das Langloch geradlinig. Gegebenenfalls das Langloch auch in Längsrichtung Bereiche unterseitlich Weite aufweisen, wobei der Bereich größter Weite vorzugsweise in der Mitte des Langloches angeordnet ist und sich das Langloch zu einem oder beiden Enden hin verjüngt. Das Langloch kann eine Verschiebung des Verbindungsmittels in Längsrichtung des Langloches in Bezug auf die bei einer Bewegung des jeweiligen Patienten anatomisch noch ermöglichten Lageveränderungen der Gelenkteile zueinander begrenzen (so dass zumindest eines oder beide Enden des Langloches als Anschlag für das Verbindungselement dienen) oder gerade nicht (also vorzugsweise keines der beiden Enden des Langloches als Anschlag für das Verbindungselement fungiert). Vorzugsweise weist das Langloch quer zu seiner Längsrichtung eine Weite auf, so dass dieses als seitliche Führung für das Verbindungselement bei einer Verschiebung des Verbindungselementes dient, vorzugsweise aber mit einer Führung mit seitlichem Spiel, so dass die laterale Weite des Langloches größer als der Durchmesser des Durchgriffsbereiches des Verbindungsmittels ist. Die erfindungsgemäße Ausbildung des Implantats hat sich bei einer derartigen Ausführung desselben als besonders vorteilhaft erwiesen, da aufgrund der ermöglichten Verschiebung der beiden Implantatteile gegeneinander in der Implantathauptebene bei einer Bewegung des Patienten Querkräfte auf die Implantatteile ausgeübt werden können, welche bei dem erfindungsgemäßen Implantat vollständig aufgefangen werden können, da die Fügerichtung von Gelenk- und Anlageteil, in welcher diese beiden Bauteile vor der Verdrehung zum Zusammenwirken der Formschlussmittel miteinander zusammenfügbar sind in einem Winkel zur Implantathauptebene liegen kann, beispielsweise in einem Winkel von ≥ ± 30 - ± 60°, besonders bevorzugt senkrecht zur Implantathauptebene. Hierdurch können Querkräfte in besonders hohem Ausmaß durch die Fügeverbindung aufgefangen werden.

Besonders bevorzugt weist das Befestigungsmittel zur Befestigung von Gelenk- und Anlageteil aneinander an zumindest einem oder beiden Implantatteilen ein männliches und ein weibliches Formschlussmittel auf, welche zur Befestigung zusammenwirken. Das männliche Formschlussmittel weist einen Zapfen, beispielsweise im Querschnitt nicht-rund, mit Haltebereichen aufweist, welche vorzugsweise radial von dem Zapfenumfang vorstehen. Das weibliche Formschlussmittel weist hierbei eine vorzugsweise nicht-runde Aufnahme mit Hinterschneidungen auf, wobei die Aufnahme ausgebildet ist, um das männliche Formschlussmittel aufzunehmen, vorzugsweise kongruent aufzunehmen. Die Formschlussmittel sind in einer Fügestellung zusammenfügbar, so dass in der Fügestellung das männliche Befestigungsmittel in der Aufnahme angeordnet ist. Unter Verdrehung der Formschlussmittel gegeneinander sind diese in eine Befestigungsstellung zur Festlegung von Gelenk- und Anlageteil aneinander überführbar. Durch die Anordnung des mindestens einen Haltebereichs in der Hinterschneidung ist eine sichere Festlegung von Gelenk- und Anlageteil aneinander gegeben. Bei der Einführung des Haltebereichs in die Hinterschneidung kann der Haltebereich auf einer Steigung in Art einer Gewindesteigung auflaufen, um hierdurch in seiner Sollposition in Axialrichtung des Zapfens zwischen den die Hinterschneidung bildenden Bereichen fixiert zu werden. Der Haltebereich kann jedoch auch passgenau in die Hinterschneidung eingepasst werden, so dass der Haltebereich axial spielfrei in der Hinterschneidung angeordnet ist. Die Formschlussmittel können somit in Art eines Bajonettverschlusses zusammenwirken.

Das Befestigungsmittel kann jeweils ein männliches und ein weibliches Formschlussmittel aufweisen, welche zur Befestigung zusammenwirken, wobei das männliche Formschlussmittel einen Zapfen mit radial vorstehenden Haltebereichen und das weibliche Formschlussmittel eine nichtrunde Aufnahme mit mindestens einer Hinterschneidung aufweist, und wobei die Formschlussmittel in einer Fügestellung zusammenfügbar und unter Verdrehung zueinander, vorzugsweise hierbei ohne axiale Verschiebung der Formschlussmittel zueinander, in eine Befestigungsstellung überführbar sind.

Vorzugsweise weisen das männliche Formschlussmittel zwei oder mehr radial vorstehende Bereiche und das weibliche Formschlussmittel mit diesen korrespondierende, eine Zusammenfügung der Formschlussmittel ermöglichende Ausnehmungen auf.

Vorzugsweise sind mindestens zwei radial vorstehende Bereiche des männlichen Formschlussmittels um mehr als 90° voneinander in Umfangsrichtung des Befestigungsmittels voneinander beabstandet angeordnet.

Vorzugsweise umfassen die männlichen und weiblichen Formschlussmittel Haltebereiche, welche in der Befestigungsstellung der Formschlussmittel Gelenk- und Anlageteil zueinander spielfrei gegen seitliche Verkippung sichern.

Besonders bevorzugt sind Verdrehsicherungsmittel vorgesehen, welche die aneinander befestigten Gelenk- und Anlageteile in ihrer Sollposition zueinander verdrehfest festlegen. Die Verdrehsicherungsmittel können an dem Bauteil von Gelenk- und Anlageteil, vorzugsweise dem Gelenkteil, an dem Zapfen vorgesehen sein, besonders bevorzugt an den radial vorstehenden Haltebereichen des Zapfens. An der mit dem Zapfen korrespondierenden Aufnahme des anderen Bauteils, vorzugsweise des Anlageteils, kann das Verdrehmittel an der Aufnahme vorgesehen sein, vorzugsweise an dem Bereich, welcher die Hinterschneidung zur axialen Festlegung der Haltebereiche des anderen Bauteils aufweist. Durch diese Verdrehsicherung sind Gelenk- und Anlageteil sicher aneinander befestigt, auch bei komplexen Bewegungen der Gelenkteile des Implantats zueinander.

Vorzugsweise sind als Rastmittel an dem Anlageteil Rastzungen angebracht sind, welche in Rastausnehmungen des Gelenkteils verdrehsichernd eingreifen, wobei die Rastmittel als Verdrehsicherungsmittel fungieren.

Besonders bevorzugt sind die Verdrehsicherungsmittel derart ausgebildet, dass diese eine Verdrehung von Gelenk- und Anlageteil zueinander in beiden Verdrehrichtungen, also sowohl bei Rechts- als auch bei Linksdrehung, gegeneinander sichern. Besonders bevorzugt sind an dem Implantatteil jeweils zumindest zwei Verdrehsicherungsmittel vorgesehen, welche das Gelenkteil und das Anlageteil jeweils in gegenläufigen Verdrehrichtungen verdrehsichern. Hierdurch können die Verdrehsicherungsmittel besonders hohe Drehmomente aufnehmen. Weiterhin können hierdurch die beiden Verdrehsicherungsmittel unabhängig voneinander gelöst und/oder betätigt werden, so dass eine Verdrehung von Gelenk- und Anlageteil gegeneinander in einer Verdrehrichtung ermöglicht und in der gegenläufigen Verdrehrichtung blockiert ist, was bei einer Demontage des Implantatteils von Vorteil sein kann. Nach einer besonders vorteilhaften Ausführungsform sind die Verdrehsicherungsmittel als Schwenkelemente oder Rastmittel ausgebildet, welche in eine korrespondierende Ausnehmung oder einen Haltebereich des korrespondieren Bauteils eingreifen, um dieses verdrehzusichern.

Vorzugsweise ist an dem Gelenkteil und/oder dem Anlageteil eines Implantatteils im Bereich der Verdrehsicherungsmittel eine außenseitig zugängliche Eingriffsöffnung für einen Stellschlüssel vorgesehen, so dass mittels des Stellschlüssels das Verdrehsicherungselement in seine Sicherungsstellung überführbar ist.

Vorzugsweise weisen eines oder beide Gelenkteile des Implantats seitliche Angriffsflächen für ein Drehmomentübertragungswerkzeug auf, welche zur Drehmomentübertragung an das Werkzeug angepasst sind, um das Gelenkteil um seine Längsachse mittels des Werkzeuges zu verdrehen.

Vorzugsweise sind im montierten Zustand des Implantats das obere und untere Implantatteil gegeneinander und/oder gegen das Zwischenelement um die Implantatlängsachse verdrehbar. Vorzugsweise sind, alternativ oder in Kombination, im montierten Zustand des Implantats das obere und untere Implantatteil gegeneinander und/oder gegen das Zwischenelement in einer Richtung quer zur Implantatlängsachse verschiebbar.

Weiterhin wird eine Montageplatte zur Montage eines erfindungsgemäßen Implantats beschrieben (nicht beansprucht), as wobei die Montageplatte mindestens eine Vertiefung zur Aufnahme eines Anlageteils aufweist. Anstelle der Vertiefung kann auch allgemein ein Haltebereich zur Halterung bzw. Aufnahme des jeweiligen Implantateils an der Montageplatte vorgesehen sein, hier für das Anlageteil, im allgemeinen für das jeweilge Implantatteil. Die Aufnahme ist hierbei vorzugsweise derart an die Umfangsgestalt des Anlageteils angepasst, dass das Anlageteil verdrehfest von der Aufnahme der Montageplatte aufgenommen wird. Hierdurch ist eine einfache Montage der Implantatteile bzw. des Implantats insgesamt ermöglicht, wobei das Anlageteil mit der Aufnahme für das Befestigungsmittel des Gelenkteils freiliegt und bei dem Verdrehen der Bauteile zueinander das Anlageteil mittels der Montageplatte auf einfache Weise gehaltert werden kann. Die Montageplatte kann mehrere Aufnahmen aufwiesen, so dass sämtliche Bauteile des Implantats separat zueinander an der Montageplatte halterbar sind. Hierdurch kann die Montageplatte mit mehreren oder vorzugsweise sämtlichen Einzelbauteilen des Implantats bestückt werden, wobei die Bauteile an die anatomischen Gegebenheiten des jeweiligen Patienten angepasst sind, so dass dann eine Kontrolle der Implantatteile erfolgen kann, beispielsweise hinsichtlich derer fehlerfreien Ausgestaltung und/oder deren geeigneter Abstimmung in Bezug auf die anatomischen Verhältnisse des Patienten.

Die erfindungsgemäße Montageplatte ist vorzugsweise zur Montage eines Implantats nach dem Oberbegriff von Anspruch 1 oder nach einem der Ansprüche 1 bis 13 ausgebildet. Vorzugsweise weist hierbei die Montageplatte mindestens eine Vertiefung zur Aufnahme eines Anlageteils und/oder eines Gelenkteils auf, welche derart an die Umfangsgestalt des zugeordneten Anlage- oder Gelenkteils angepasst ist, dass das jeweilige Anlage- oder Gelenkteil in Bezug auf die Montageplattenhauptebene verschiebungssicher und in Bezug auf die Hauptachse von Anlage- oder Gelenkteil verdrehfest von der Aufnahme der Montageplatte aufgenommen ist. Vorzugsweise ist die Montageplatte jeweils mit den in der Beschreibung und/oder in den Ansprüchen genannten Implantatteilen bestückt, insbesondere jeweils mit den in einem Anspruch zu der Montageplatte genannten Implantatteilen.

Vorzugsweise ist an der Montageplatte ein in einer Aufnahme anordenbarer Einsatz vorgesehen ist, welcher an die Umfangskontur und/oder Oberflächenstruktur des in der Aufnahme anzuordnenden Implantateils angepasst ist.

Weiter beschrieben (nicht beansprucht) ist ein Montageset umfassend eine Montageplatte, insbesondere bevorzugt jeweils bestückt mit sämtlichen Implantatteilen, für welche zugeordnete Vertiefungen vorgesehen sind, und einen Stellschlüssel, mittels welchem zumindest einer der Verdrehsicherungsmittel in seine verdrehsichernde Stellung an dem Gelenkteil überführbar ist.

Weiter beschrieben (nicht beansprucht) ist ein Montageset umfassend eine Montageplatte, insbesondere bevorzugt jeweils bestückt mit sämtlichen Implantatteilen, für welche zugeordnete Vertiefungen vorgesehen sind, und mit einem Drehmomentübertragungsschlüssel zur verdrehenden Befestigung eines Gelenkteils an einem Anlageteil des Implantats. Ferner wird beschrieben ein Montageset umfassend eine Montageplatte, insbesondere bevorzugt jeweils bestückt mit sämtlichen Implantatteilen, für welche zugeordnete Vertiefungen vorgesehen sind, und einen Stellschlüssel, mittels welchem zumindest einer der Verdrehsicherungsmittel in seine verdrehsichernde Stellung an dem Gelenkteil überführbar ist und mit einem Drehmomentübertragungsschlüssel zur verdrehenden Befestigung eines Gelenkteils an einem Anlageteil des Implantats.

Die Erfindung wird nachfolgend beispielhaft beschrieben und anhand der Figuren erläutert. Es zeigen:
Figur 1: Ein erfindungsgemäßes Implantat in Explosionsdarstellung (Fig. 1a) und in montiertem Zustand im Querschnitt (Fig. 1b),
Figur 2: Eine Explosionsdarstellung der Baugruppe der beiden Gelenkteile mit Verbindungsmittel gemäß dem Implantat nach Figur 1 (Fig. 2a) und im montierten Zustand (Fig. 2b),
Figur 3: Eine Anordnung aus Anlageteil und Gelenkteil in Explosionsdarstellung (Fig. 3a) und im Fügezustand (Fig. 3b) sowie im verrasteten Zustand (Fig. 3b) in Draufsicht,
Figur 4a-f: Eine Montageplatte mit Teilen des Implantats in perspektivischer Ansicht bei der Teilmontage eines Implantats.

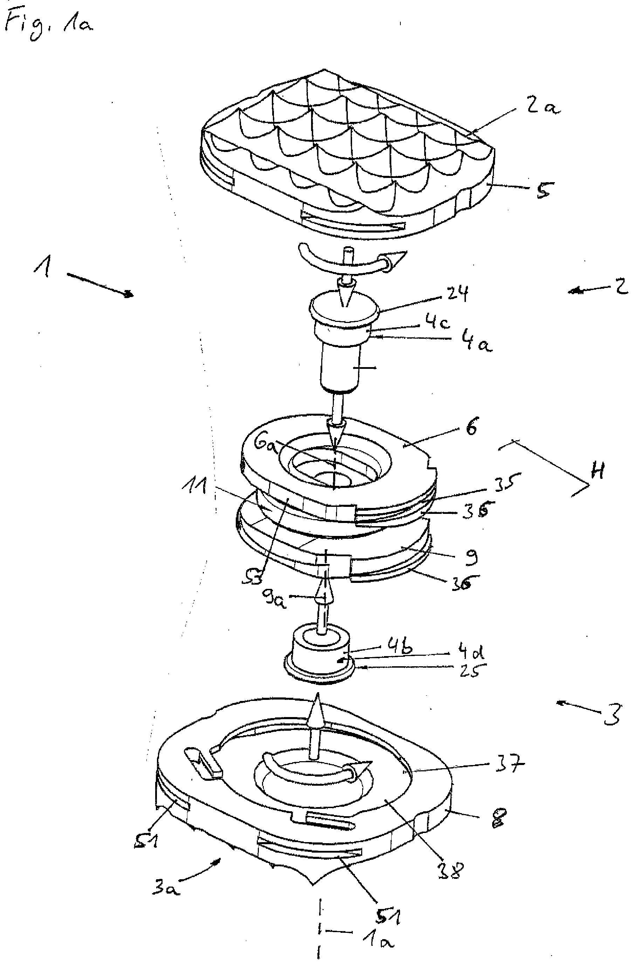

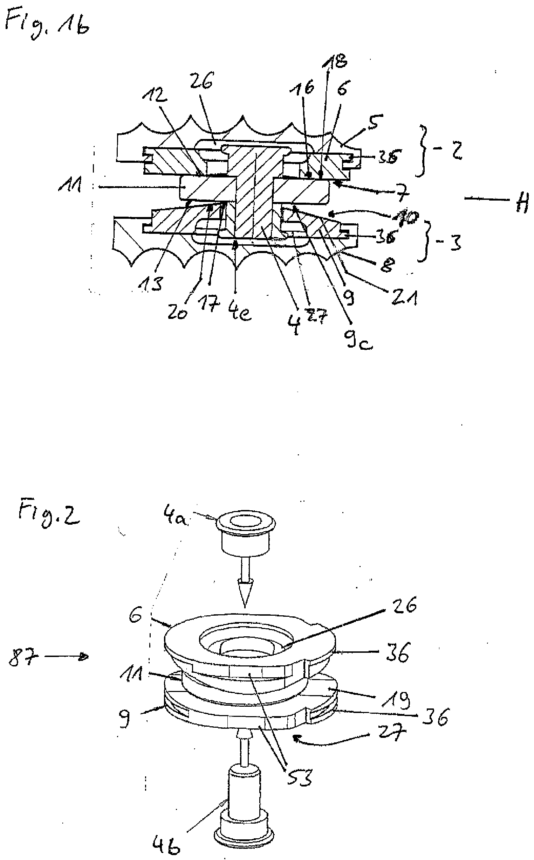

Die Figuren 1-3 zeigen eine Ausführungsform eines erfindungsgemäßen Gelenkimplantats 1 mit einem oberen und einem unteren Implantatteil 2, 3 und mit einer Implantatlängsachse 1a, wobei das obere Implantatteil 2 mit einer Oberseite 2a und das untere Implantatteil 3 mit seiner Unterseite 3a jeweils an die Anlagebereiche von Knochen, insbesondere die Grundplatten angrenzender Wirbelkörper, anlegbar sind, um diese gelenkig miteinander zu verbinden. Das Anlageteil weist eine dem zugeordneten Knochen zugewandte durchgehend geschlossene Oberfläche auf. Das obere Implantatteil ist mit seiner Oberseite und das untere Implantatteil mit seiner Unterseite jeweils an gelenkig miteinander zu verbindenden Knochen anlegbar und abstützbar.

Weiterhin ist ein Verbindungsmittel 4 vorgesehen, welches die beiden Implantatteile 2,3 unter Ausbildung einer zusammenhängenden Baugruppe unter Ermöglichung einer Gelenkbewegung derselben miteinander verbindet. Das obere Implantatteil 2 besteht hierbei aus einem Anlageteil 5, welches die an dem Knochen anlegbare Oberseite 2a des Implantatteils aufweist, und einem an dem Anlageteil 5 befestigten, vorzugsweise separaten Gelenkteil 6, welches den Gelenkbereich 7 des oberen Implantatteils bereitstellt. Das untere Implantatteil 3 weist entsprechend ein die Unterseite 3a bereit stellendes Anlageteil 8 sowie ein an diesem befestigtes, vorzugsweise separates Gelenkteil 9 auf, welches den Gelenkbereich 10 des unteren Implantatteils bereit stellt. Gelenk- und Anlageteil des jeweiligen Implantatteils durch Befestigungsmittel lösbar miteinander verbunden, die Verbindung der Implantat- und Gelenkteile miteinander ist jeweils lösbar. Die beiden Anlageteile und die beiden Gelenkteile des Implantats des Ausführungsbeispiels sind baugleich, Fig. 1 jedoch um 90° zueinander verdreht dargestellt. Die beiden Anlageteile und die beiden Gelenkteile des Implantats können jeweils unabhängig voneinander auch unterschiedlich ausgeführt sein, insbesondere mit unterschiedlichen Gelenkbereichen der beiden Gelenkteile, z.B. unterschiedliche Wölbungen und/oder Breiten der Rücken und/oder unterschiedliche Neigungen der vorzugsweise ebenen, sich lateral an die Rücken anschließenden Bereiche, welche durch Anlage an dem Zwischenelement die Abwälzbewegung begrenzen. Die Ausführungen nach diesem Ausführungsbeispiel können allgemein jeweils für nur eines der beiden Implantatteile des erfindungsgemäßen Implantats gelten, z.B. für das obere oder das unteren Implantatteil, insbesondere gelten sie für beide Implantatteile des Implantats.

Die beiden Gelenkbereiche 7,10 des oberen und unteren Implantatteils können ggf. unmittelbar miteinander unter Ausbildung einer Gelenkverbindung zusammenwirken. Nach dem Ausführungsbeispiel ist zwischen den beiden Implantatteilen 2,3 jedoch ein Zwischenelement 11 vorgesehen, welches eine dem oberen und unteren Anlageteil 5,8 zugewandte Ober- und Unterseite 12,13 aufweist, welche jeweils mit den Gelenkoberflächen 16,17 der Gelenkteile 6,9 unter Anlage an diese zusammenwirken, um so zwei Teilgelenke des Implantats auszubilden. Die beiden Gelenkbereiche 7,10 bzw. die beiden Teilgelenke sind hierbei unabhängig voneinander betätigbar bzw. bewegbar. Das Verbindungsmittel 4 verbindet das obere und untere Implantatteil 2,3 unter Ausbildung einer zusammenhängenden Baugruppe vorzugsweise unlösbar miteinander. Das Verbindungsmittel 4 ist gegenüber dem oberen und/oder unteren Gelenkteil 6,9 lageveränderlich, vorzugsweise zumindest gegenüber einem derselben. Das Verbindungsmittel ist hier gegenüber dem oberen und/oder unteren Gelenkteil um dessen Längsachse 6a, 9a verdrehbar und vorzugsweise zumindest in einzelnen Gelenkstellungen des Implantats (vorzugsweise auch der Neutralstellung) geringfügig gegen diese axial verschiebbar. Das Verbindungsteil kann unabhängig oder in Kombination hiermit lageveränderlich zu dem Zwischenteil sein, beispielsweise gegenüber diesem verdrehbar und/oder in der Implantathauptebene verschiebbar sein, vorzugsweise ist das Verbindungsmittel 4 zu dem Zwischenteil 11 aber lagefixiert. Das Verbindungsmittel kann gegenüber den Gelenkteilen 6, 9 in der Implantathauptebene H (welche senkrecht zu der Achse 1a steht) verschiebbar sein, in zumindest einer oder vorzugsweise in genau einer Richtung, welche durch ein Langloch 6b, 9b des jeweiligen Gelenkteils vorgegeben sein kann.

Die Ober- und/oder Unterseite 12,13 des Zwischenelements 11, vorzugsweise beide, sind hier eben ausgeführt und vorzugsweise planparallel zueinander angeordnet. Die Gelenkoberflächen 16, 17 können jeweils gegenüber den Oberflächen 12,13 des Zwischenelementes 11 unter Ausbildung von Teilgelenken lageveränderlich sein, insbesondere in einer Abwälzbewegung (unter Umständen und deutlich weniger praktikabel auch eine Verschwenk- oder Verkippbewegung). Die Achse, um welche jeweils die Abwälzbewegung durchgeführt wird, kann zu der Implantatlängsachse einen Winkel von 90° einschließen. Zur Durchführung der Abwälzbewegung sind die Gelenkoberflächen 16,17 jeweils mit einem mittigen gewölbten, vorzugsweise kreisbogenförmig gewölbten, Abwälzbereich in Form eines Rückens versehen, welcher jeweils zum Zwischenelement vorsteht. "Abwälzbewegung" heißt, dass sich die in Neutralstellung des Implantats an dem Zwischenelement anliegende Linie des Gelenkbereichs des Gelenkteils, also der Grat des Rückens, bei der Abwälzung von der Anlagefläche des Zwischenelementes abhebt, also durch einen Spalt 9c (entsprechend auch beim Gelenkteil 6, hier von dem Gelenkteil verdeckt) von dem Zwischenelement getrennt ist (im Unterschied zur Ausbildung eines Kugelgelenkes). Dies ist für eine vorteilhafte anatomische Ausbildung des Implantats von wesentlicher Bedeutung. Seitlich an die die Abwälzbewegung ermöglichenden Bereiche, hier die als Abwälzbereiche ausgebildeten Gelenkbereiche 7, 10, schließen sich jeweils vorzugsweise eben ausgebildete Anlagebereiche 20,21 an, die an dem oberen (nicht dargestellt) und unteren Gelenkteil vorgesehen sind, und die vorzugsweise eine flächige Anlage an die Ober- und Unterseite des Zwischenelements ermöglichen und zugleich die Gelenkbewegung begrenzen. Die Anlagebereiche 20,21 des jeweils oberen und unteren Implantatteil können in der Neutralstellung dieser Teile, d.h. bei senkrecht zur Implantatlängsachse angeordneten Anlageflächen, jeweils dieselbe oder eine unterschiedliche Neigung zu der Implantatlängsachse la aufweisen, welche zugleich mit der Längsachse des Verbindungsmittels 4 übereinstimmen kann. Das obere und untere Gelenkteil können sich hierbei im Hinblick auf die Ausbildung der Gelenkbereiche unterscheiden, beispielsweise im Hinblick auf die Wölbungs- oder Krümmungsradien der mittleren Abwälzbereiche und/oder die Neigung der sich seitlich anschließenden Anlagebereiche 20,21 zur Implantatlängsachse. Die Ausgestaltung erfolgt gemäß den anatomischen Erfordernissen. Das obere und untere Anlageteil 5, 8 können allgemein und unabhängig von dem Ausführungsbeispiel baugleich ausgeführt sein. Die einzelnen Bauteile, d.h. das obere und untere Anlageteil 5, 8, das obere und untere Gelenkteil 6,9 und das Zwischenelement 11 können im Wesentlichen als plattige Bauteile ausgebildet sein. Das obere und untere Implantatteil 2,3 sind gegeneinander und gegen das Zwischenelement um die Implantatlängsachse 1a verdrehbar ausgebildet.

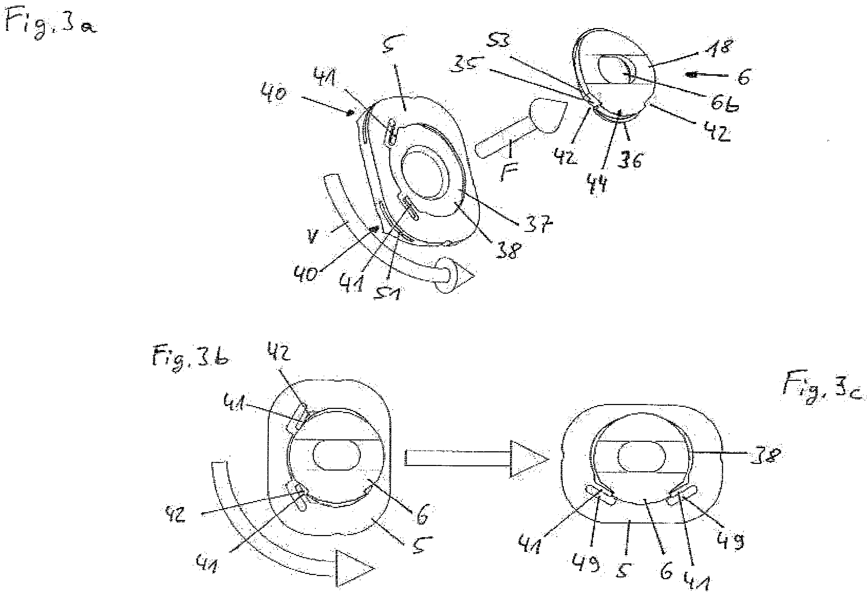

Bei der Gelenkbewegung kann zugleich eine Art "Durchrutschen" des Abwälzbereiches in Abwälzrichtung über die jeweilige Oberfläche des Zwischenelements 11 erfolgen, was dadurch bewirkt werden kann, dass die inneren Seitenwände der Verbindungsmittelaufnahme (bzw. die die Langlöcher seitlich begrenzenden Seitenwände der Aufnahme; siehe Bezugsziffer 30, Fig. 2, 3, entsprechend beim oberen Implantatteil um 90° verdreht) bei einer Verschwenk- bzw. Abwälzbewegung des Anlageteils gegenüber dem Zwischenelement mit dem Verbindungsmittel 4 zur Anlage kommen. Die Rutschbewegung kann sich vorzugsweise an die Abwälzbewegung anschließen, so dass diese beiden Bewegungen zeitlich nacheinander, also nicht überlagert, erfolgen. Nach dem Ausführungsbeispiel sind die Breite des Langloches und die Gelenkfläche des Gelenkteils derart ausgeführt, dass zunächst eine Abwälz- und dann anschließend eine Rutschbewegung erfolgt, was für beide Gelenkteile gelten kann. Auch bei der "Rutschbewegung" liegt das Gelenkteile linienförmig (und nicht flächig) an der jeweiligen Anlagefläche des Zwischenelementes an.

Das Verbindungsmittel 4 ist nach dem Ausführungsbeispiel zweiteilig mit zwei zusammenfügbaren Teilen ausgebildet, beispielsweise mit einem Bolzen 4a und einem Aufnahmezapfen bzw. Aufnahmehülse 4b. Das Verbindungselement ist gegenüber dem Zwischenelement axial spielfrei gesichert, vorzugsweise auch verdrehfest gesichert, wozu die Teile 4a,4b im befestigten Zustand jeweils mit ihren kranzförmigen Anlagebereichen 4c, 4d die Ober- und Unterseite 12,13 des Zwischenelements 11 übergreifen und spielfrei an diesen anliegen. Der Bolzen 4a und die Aufnahmehülse 4b sind dauerhaft miteinander verbunden (vorzugsweise nicht zerstörungsfrei lösbar), beispielsweise an dem stirnseitigen Verbindungsbereich 4e (siehe Fig. 2), beispielsweise durch Laserschweißen. Die Verbindung kann bei zumindest teilweise oder vollständig demontiertem Anlageteil (vorzugsweise bei beiden noch nicht montierten Anlageteilen) erfolgen, die Verbindung kann zumindest im Wesentlichen aus der Längsrichtung des Verbindungsmittels bzw. der Implantatlängsachse her erfolgen. Durch die Verbindung wird eine zusammenhängende Unterbaugruppe aus Gelenkteilen, Zwischenelement und Verbindungselement erzeugt. Die kopfartig erweiterten Verbindungsbereiche 24,25 des Verbindungsmittels 4 sind hierbei in Aufnahmen 26,27 der beiden Gelenkteile angeordnet und übergreifen die Haltebereiche der Gelenkteile, um die beiden Gelenkteile zusammen mit dem dazwischen angeordneten Zwischenelement axial aneinander zu sichern.

Die Aufnahme zumindest eines, nach dem Ausführungsbeispiel die Aufnahmen beider Gelenkteile, sind mit Langlöchern 6b,9b versehen, die eine laterale Verschiebung der beiden Implantatteile 2,3 quer zu der Verbindungsmittellängsachse gegenüber dem Verbindungselement ermöglichen. Die Langlöcher erstrecken sich hier in Längsrichtung der die Gelenkbewegung ermöglichenden Bereiche 18, 19 (die hier als Abwälzbereiche ausgeführt sind), also der Rücken der Gelenkteile, und zwar auf Höhe der Rückenlinie (Linie der höchsten Erhebung). Die Rückenlinie des jeweiligen Gelenkteils verläuft in der Mittelinie des jeweiligen Langloches. Das Gelenkteil des oberen und unteren Implantatteils ist somit jeweils als Gleitplatte ausgebildet, welche durch Verschiebung entlang des jeweiligen Langloches auf dem Zwischenelement gleitend bewegt wird. Diese Gleit- oder Verschiebungsbewegung wirkt somit nicht in Richtung einer Demontagebewegung eines der Paare von Gelenk- und Anlageteil.

Die Anlageteile 5,8 sind jeweils lösbar mit den zugeordneten Gelenkteilen 6,9 verbunden, hier jeweils durch Formschlussmittel, insbesondere durch Rastmittel, wie in Fig. 3 für Paarung von Anlage- und Gelenkteil dargestellt (entsprechendes gilt für das Implantatteil 3). Die andere Paarung der beiden Teile ist in Bezug auf die Formschlussmittel vorzugsweise baugleich ausgeführt. Erfindungsgemäß sind die Befestigungsmittel von Gelenk- und Anlageteil des jeweiligen Implantatteils unter Verdrehung der beiden Teile zueinander in Wirkeingriff bringbar, und zwar unter Verdrehung um die Implantatlängsachse 1a bzw. die Längsachsen 6a,9a der Implantatteile. Hierdurch sind, wie festgestellt wurde, bei einer gelenkigen Bewegung des Implantats die Formschlussmittel in Bezug auf eine Bewegung in Lösungsrichtung derselben zumeist außerhalb der Hauptkraftflusslinien, insbesondere von solchen die in der Implantathauptebene verlaufen, so dass einer unbeabsichtigten Lösung der Formschlussmittel entgegengewirkt wird. Die Befestigungsmittel sind derart ausgeführt, dass Gelenk- und Anlageteil in Richtung der Implantatlängsachse 1a zusammenzufügen sind, um diese in einer ersten Eingriffsstellung oder Fügeposition miteinander zum Eingriff zu bringen (siehe Fig. 3b) und dann ausgehend von dieser Stellung durch Verdrehung in ihre Befestigungsstellung 8siehe Fig. 3c) zu überführen. Anlage- und Gelenkteil sind hierbei durch die Haltebereiche 36 am Gelenkteil in Axialrichtung der Implantatlängsachse formschlüssig gesichert. Hierdurch kann die Zusammenfügung einfach bewerkstelligt werden. Die Verdrehung zur Festlegung mittels der Rastmittel erfolgt also auch um die Fügerichtung (Pfeil F), welche parallel der Längsachsen 6a,9a verläuft.

Das Befestigungsmittel weist jeweils ein männliches und ein zusammenwirkendes weibliches Formschlussmittel auf, wobei das männliche Formschlussmittel einen Zapfen 35 oder Sockel mit hier zwei radial vorstehenden Haltebereichen 36 und das weibliche Formschlussmittel eine nichtrunde Aufnahme 37 mit mindestens einer Hinterschneidung aufweist, wobei die Aufnahme 37 der Aufnahme des Anlageteils für das Gelenkelement dient und laterale Ausbauchungen 38 zur Einführung des Haltebereiche 36 aufweist. Die Aufnahme 37 ist somit insgesamt nicht-rund ausgeführt. Hierdurch ist die Zusammenfügung einfach durchführbar und kann hohe Kräfte aufnehmen, so dass das Implantat eine lange Lebensdauer aufweist. Die Haltemittel 36 einerseits und die Ausbauchungen 38 andererseits sind zueinander in der Form (bezogen auf deren Draufsicht) kongruent ausgeführt. Der Befestigungsbereich des Anlageteils für das Gelenkteil weist somit eine Aufnahme 37 auf, in welche das Gelenkteil von der bei montiertem Implantat dem Anlageteil zugewandten Fläche einführbar ist, wodurch das Implantat leicht montierbar ist. Die Formschlussmittel sind in einer Fügestellung zusammenfügbar und unter Verdrehung zueinander in eine Befestigungsstellung überführbar. Die Formschlussmittel sind derart ausgebildet, dass diese bei der Verdrehung ohne axiale Verschiebung der Formschlussmittel zueinander aus der Eingriffsstellung/Fügeposition in die Befestigungsstellung überführbar sind. Die Wirkflächen der Formschlussmittel weisen somit keine Ganghöhe auf. Hierdurch kann das Implantat eine besonders geringe Bauhöhe aufweisen und die bei einer gelenkigen Bewegung des Implantats auf die Implantatteile wirkenden Kräfte sind gut berechenbar und beherrschbar. Das männliche Formschlussmittel weist nach dem Ausführungsbeispiel zwei oder mehr radial vorstehende Bereiche (Haltebereiche 36) und das weibliche Formschlussmittel mit diesen korrespondierende, eine Zusammenfügung der Formschlussmittel ermöglichende Ausnehmungen 38 auf, wodurch eine stabile und verkippsichere Ausführungsform geschaffen ist. Die mindestens zwei radial vorstehenden Bereiche des männlichen Formschlussmittels sind hierzu nach dem Ausführungsbeispiel um mehr als 90° voneinander in Umfangsrichtung des Befestigungsmittels voneinander beabstandet angeordnet, hier um 180° beabstandet. Wie die beiden Haltemittel sind auch die beiden Ausbauchungen einander gegenüberliegend angeordnet.

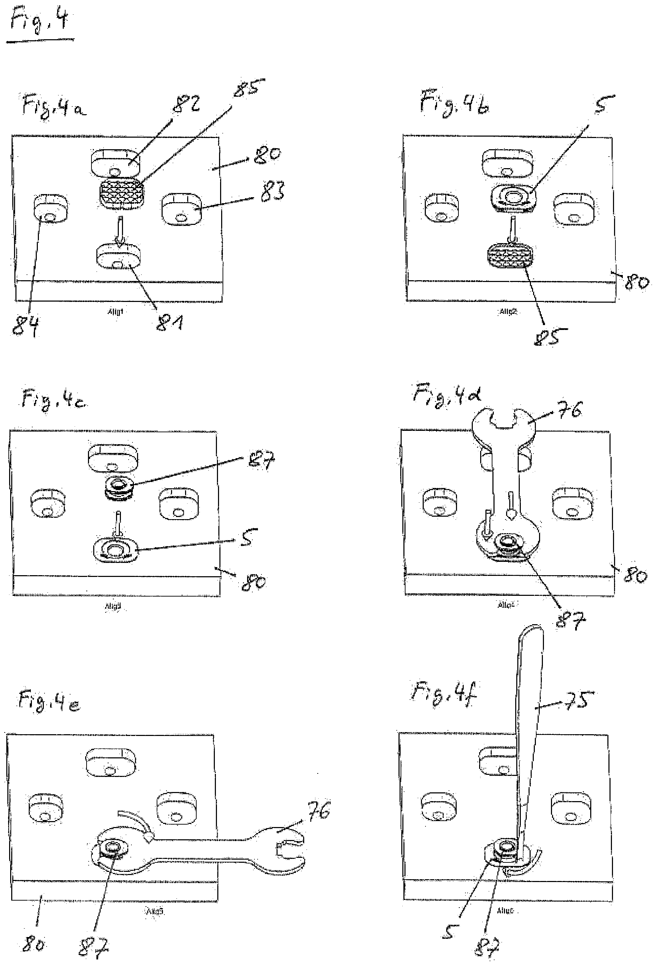

Die zusammenwirkenden Formschlussmittel sind in einer Verdrehstellung durch die Verdrehsicherungsmittel 40 gegeneinander verdrehgesichert (siehe Fig. 3), wodurch eine unbeabsichtigte Demontage des Implantats verhindert wird. Die Verdrehsicherungsmittel 40 weisen Rastmittel 41 auf, welche einer unbeabsichtigten Demontage sicher entgegenwirken und hohe Haltekräfte ermöglichen, andererseits mittels eines Stellschlüssels 75 (Fig. 4f) lageveränderlich ausgebildet sind, z.B. durch Verdrehung desselben aus einer Sicherungsstellung in eine Entsicherungsstellung überführbar sind. Die zungenförmigen Rastmittel 41 können in korrespondierende Rastausnehmungen 42 des Gelenkteils verdrehsichernd eingreifen und in entgegen gesetzten Richtungen gegen Verdrehung sperren. Hierdurch ist das Implantat bei Bedarf, beispielsweise um in einer anderen Kombination von Anlage- und Gelenkteilen erneut montiert zu werden, besonders vorteilhaft angepasst. An dem Implantatteil sind hier zwei Verdrehsicherungsmittel 40 vorgesehen, welche das Gelenkteil und das Anlageteil in gegenläufigen Verdrehrichtungen zueinander verdrehsichern, so dass eine Verdrehsicherung in beiden Verdrehrichtungen gegeben ist, mit maximaler Haltekraft in jeder der beiden Richtungen.

Die in entgegen gesetzten Drehrichtungen sperrenden Verdrehsicherungsmittel 40 sind derart ausgebildet, dass das jeweils eine Sicherungsmittel zugleich als die Drehbewegung des Gelenkteils begrenzender Anschlag fungiert. Dann, wenn das Gelenkteil an einem einen Anschlag in die jeweilige Drehrichtung ausbildendem Sicherungsmittel anliegt greift das in die andere Drehrichtung sichernde Sicherungsmittel in dem diesem zugeordneten Haltebereich, z.B. eine Rastausnehmung 42, ein und sichert das Gelenkteil gegen Verdrehen in die andere Drehrichtung, so dass das Gelenkteil dann in beiden Drehrichtungen gegenüber dem Anlageteil verdrehgesichert ist. Dies kann allgemein im Rahmen der Erfindung gelten.

Die Befestigungsmittel (z.B. Zapfen und Aufnahme) weisen jeweils eine Längsachse auf (parallel zu den Achsen 6a,9a), wobei an dem Gelenk- und/oder dem Anlageteil Bereiche mit größerem und mit kleinerem radialen Abstand von der Längsachse vorgesehen sind. Die Verdrehsicherungsmittel sind hierbei an dem Gelenkteil an den Bereichen 44 mit größerem radialen Abstand von der Längsachse angeordnet, wodurch die Verdrehsicherung höhere Kräfte aufnehmen und exakter in Bezug eine Verhinderung kleiner Verdrehbewegungen einstellbar ist.

Die männlichen und weiblichen Formschlussmittel umfassen Haltebereiche 36 auf, welche in der Befestigungsstellung der Formschlussmittel Gelenk- und Anlageteil 6,9 zueinander spielfrei gegen seitliche Verkippung sichern, wodurch die Stabilität des Implantats bei komplexen Bewegungen des Patienten erhöht wird.

An dem Gelenkteil 6 und/oder dem Anlageteil 9 eines Implantatteils ist im Bereich der Verdrehsicherungsmittel eine außenseitig zugängliche Eingriffsöffnung 49 für einen Stellschlüssel 75 vorgesehen (Fig. 1,3), mittels dessen durch Angriff an dem Verdrehsicherungselement dieses in seine Sicherungsstellung überführbar ist. Die Eingriffsöffnung dient hier zugleich, um eine Auslenkung des Rastmittels nach radial außen vor der Einrastung zu ermöglichen.

Das Anlage- oder Gelenkteil weisen an seiner umlaufenden Seitenfläche Durchgangsöffnungen 51 aufweist, welche mit der Aufnahme für das jeweils andere der beiden genannten Teile in Verbindung stehen, insbesondere dem Bereich, in welchem die Befestigungsmittel für das Gelenkteil angeordnet sind, so dass durch Einführen eines Werkzeuges wie eines Dornes oder des des Stellschlüssels 75 in die Durchgangsöffnungen die Verdrehsicherungsmittel entsperrbar sind, um eine Verdrehung der durch die Verdrehsicherungsmittel arretierten Teile und damit Demontage des Implantats zu ermöglichen.

Um eine Montage des Implantats zu erleichtern weisen eines oder beide Gelenkteile des Implantats seitliche Angriffsflächen 53 für ein Drehmomentübertragungswerkzeug 76 auf, welche zur Drehmomentübertragung an das Werkzeug angepasst sind, um das Gelenkteil um seine Längsachse mittels des Werkzeuges zu verdrehen.

Ferner ist eine Montageplatte 80 zur Montage eines Implantats nach dem Oberbegriff von Anspruch 1, vorzugsweise nach einem Implantat gemäß der Erfindung vorgesehen. Die Montageplatte weist mindestens eine Vertiefung 81 zur Aufnahme eines Anlageteils und/oder eines Gelenkteils aufweist. Die Vertiefung ist derart an die Umfangsgestalt des zugeordneten Anlage- oder Gelenkteils angepasst, dass das jeweilige Anlage- oder Gelenkteil in Bezug auf die Montageplattenhauptebene verschiebungssicher und in Bezug auf die Hauptachse von Anlage- oder Gelenkteil verdrehfest von der Aufnahme der Montageplatte aufgenommen ist. Die Montage des Implantats wird hierdurch wesentlich erleichtert, da bei der Montage das jeweilige Teil an der Montageplatte positioniert, d.h. in der Aufnahme angeordnet montierbar ist, die Montageplatte also zur Fixierung des jeweiligen Teils bei der Montage dient. Hierzu kann ferner ein in einer Aufnahme anordenbarer Einsatz 85 vorgesehen sein, welcher an die Umfangskontur und/oder Oberflächenstruktur des in der Aufnahme anzuordnenden Implantateils vorzugsweise passgenau angepasst ist.

Die Montageplatte kann mindestens zwei Aufnahmen 81, 82 zur Aufnahme beider Anlageteile des Implantats aufweist. Die Montageplatte kann mindestens oder genau vier Aufnahmen 81-85 zur Aufnahme beider Anlageteile 5,8 und beider Gelenkteile 6,9 des Implantats aufweisen. Alternativ kann die Montageplatte mindestens oder genau fünf oder sechs Aufnahmen zur Aufnahme beider Anlageteile, beider Gelenkteile und des Zwischenelementes 11 oder des Verbindungselementes 4 oder zur Aufnahme von Zwischenelement 11 und Verbindungselement 4 aufweisen. Alternativ kann die Montageplatte mindestens oder genau fünf oder sechs Aufnahmen zur Aufnahme beider Anlageteile, beider Gelenkteile, des Zwischenelementes und eines zweiteiligen Verbindungselementes aufweisen. Hierdurch können zugleich die wichtigsten oder sämtliche Bauteile des Implantats an der Montageplatte angeordnet und gehaltert werden, bspw. zur Kontrolle der Bauteile vor Montage des Implantats. Die Aufnahme kann jeweils als Vertiefung der Montageplatte oder als vorstehende Befestigungsvorsprünge oder auf andere geeignete Weise, welche vorzugsweise eine verschiebungssichere Positionierung der Teile an der Montageplatte ermöglichen, ausgebildet sein. Es versteht sich, dass die Aufnahmen der Montageplatte jeweils an die aufzunehmenden Bauteile, insbesondere Anlage- und/oder Gelenkteil angepasst sind, ggf. unter Anordnung eines jeweiligen Einsatzes 85, wobei die Bauteile jeweils verschiebungssicher, vorzugsweise passgenau, in der Aufnahme angeordnet sind. Das Montageset umfassend die Montageplatte 80 kann einen Stellschlüssel 75, mittels welchem zumindest einer der Verdrehsicherungsmittel in seine verdrehsichernde Stellung an dem Gelenkteil überführbar ist, umfassen.

Zur Montage kann (siehe Fig. 4a-f) eine Anlageplatte, die kraniale (dargestellt) oder kaudale, an der Montageplattenaufnahme verdrehfest positioniert werden, mit dem Befestigungsbereich für das korrespondierende Gelenkteil nach oben gerichtet. Aus den beiden Gelenkteilen und dem Zwischenelement ist mittels des Verbindungselementes eine Baugruppe 87 (siehe Fig. 2) implantationsfertig vormontiert. Das korrespondierende Gelenkteil wird mit seinem Befestigungsbereich in die Anlageteilaufnahme eingesetzt und mittels des Werkzeuges gegenüber dem Anlageteil verdreht, bis dieses verrastet. Der Befestigungsbereich des zweiten Gelenkteils steht dann frei nach oben vor. Das zweite Anlageteil wird dann befestigt, um das Implantat fertigzustellen. Die insoweit vormontierte Baugruppe wird vorzugsweise der Montageplattenaufnahme entnommen und der Befestigungsbereich des zweiten Gelenkteils in die Aufnahme des in seiner zugeordneten Montageplattenaufnahme positionierten Anlageteils eingesetzt, das zweite Gelenkteil gegenüber dem zweiten Anlageteil mittels des Werkzeuges verdreht und ebenfalls an dem Anlageteil verrastet. Gegebenenfalls kann auch das zweite Anlageteil auf die vormontierte Baugruppe aufgesetzt, und das Gelenkteil gegen das Anlageteile verdreht werden, was jedoch umständlicher und fehleranfälliger ist. Ist das jeweilige Gelenkteil an dem korrespondierenden Anlageteil in seiner Befestigungsstellung, so kann durch Einführung des Stellschlüssels in die Eingriffsöffnung die ordnungsgemäße Positionierung der Verdrehsicherungsmittel überprüft bzw. sichergestellt werden.

## Patentansprüche

1. Gelenkimplantat mit einem oberen und einem unteren Implantatteil (2,3), welche unter Ausbildung eines Gelenkes gelenkig miteinander verbunden sind, und mit einer Implantatlängsachse (1a), wobei das obere und das untere Implantatteil (2,3) jeweils ein den Gelenkbereich umfassendes Gelenkteil (6,9) und ein an dem jeweils zugewandten Knochen anlegbares und abstützbares Anlageteil (5,8) aufweist, wobei Gelenkteil (6,9) und Anlageteil (5,8) des jeweiligen Implantatteils (2,3) durch Befestigungsmittel lösbar miteinander verbunden sind, wobei die beiden Gelenkteile (6,9) der Implantatteile (2,3) jeweils einen Gelenkbereich (7,10) aufweisen und die beiden Gelenkbereiche (7,10) mit einem Zwischenelement (11) mit gegenüberliegender Ober - und Unterseite (12,13) als Anlageflächen unter Ausbildung von zwei Teilgelenken zusammenwirken, und wobei ein Verbindungsmittel (4) vorgesehen ist, welches das obere und untere Implantatteil (2,3) unter Ausbildung einer zusammenhängenden Baugruppe miteinander verbindet, wobei das Verbindungsmittel (4) lageveränderlich zu dem oberen und unteren Gelenkteil (6,9) ist, **dadurch gekennzeichnet, dass** die beiden Implantatteile (2,3) gegeneinander und gegen das Zwischenelement (11) frei verdrehbar sind, dass die Befestigungsmittel von Anlage- und Gelenkteil (5, 8; 6, 9) als Formschlussmittel (35,36;37) ausgebildet sind, die unter Verdrehung von Gelenkteil (6,9) und Anlageteil (5,8) des jeweiligen Implantatteils (2,3) aneinander befestigbar bzw. befestigt sind, dass die zusammenwirkenden Formschlussmittel (35,36;37) in einer Verdrehstellung durch Verdrehsicherungsmittel (41,42) gegeneinander verdrehgesichert sind und dass die Verdrehsicherungsmittel Rastzungen (41) aufweisen.

2. Implantat nach Anspruch 1, **dadurch gekennzeichnet, dass** die Formschlussmittel (35,36;37) derart ausgebildet sind, dass diese in Implantatlängsrichtung unter Überführung in eine Fügeposition zusammenfügbar sind, und in der Fügeposition durch Verdrehung gegeneinander in Bezug auf die Fügerichtung unter Formschlussbildung aneinander befestigbar sind.

3. Implantat nach Anspruch 1 oder 2, **dadurch gekennzeichnet, dass** das Befestigungsmittel jeweils ein männliches und ein weibliches Formschlussmittel (35,36;37) aufweist, welche zur Befestigung zusammenwirken, und dass das männliche Formschlussmittel einen Zapfen (35) mit radial vorstehenden Haltebereichen (36) und das weibliche Formschlussmittel (37) eine nichtrunde Aufnahme mit mindestens einer Hinterschneidung aufweist, und dass die Formschlussmittel (35,36;37) in einer Fügestellung zusammenfügbar und unter Verdrehung zueinander in eine Befestigungsstellung überführbar sind.

4. Implantat nach einem der Ansprüche1 bis 3, **dadurch gekennzeichnet, dass** die Verdrehsicherungsmittel Rastmittel (41,42) aufweisen oder mittels eines Stellschlüssels lageveränderliche Formschlussmittel sind.

5. Implantat nach Anspruch 4, **dadurch gekennzeichnet, dass** an einem Implantatteil (2,3) zwei Verdrehsicherungsmittel (41,42) vorgesehen sind, welche das Gelenkteil (6,9) und das Anlageteil (5,8) in gegenläufigen Verdrehrichtungen zueinander verdrehsichern.

6. Implantat nach einem der Ansprüche 1 bis 5, **dadurch gekennzeichnet, dass** die als Formschlussmittel (35,36;37) ausgebildeten Befestigungsmittel jeweils eine Längsachse aufweisen und dass an dem Gelenk- und/oder dem Anlageteil (5,8;6,9) Bereiche mit größerem und mit kleinerem radialen Abstand von der Längsachse vorgesehen sind und dass die Verdrehsicherungsmittel (41,42) an den Bereichen mit größerem radialen Abstand von der Längsachse angeordnet sind.

7. Implantat nach einem der Ansprüche 1 bis 6, **dadurch gekennzeichnet, dass** der Befestigungsbereich des Anlageteils (5,8) für das Gelenkteil (6,9) eine Aufnahme aufweist, in welche das Gelenkteil von der bei montiertem Implantat dem Anlageteil zugewandten Fläche in die Aufnahme einführbar ist.

8. Implantat nach einem der Ansprüche 1 bis 7, **dadurch gekennzeichnet, dass** das Anlage- oder Gelenkteil (5,8;6,9) an seiner umlaufenden Seitenfläche Durchgangsöffnungen (51) aufweist, welche mit der Aufnahme für das jeweils andere der beiden genannten Teile in Verbindung stehen.

9. Implantat nach einem der Ansprüche 1 bis 8, **dadurch gekennzeichnet, dass** das Verbindungsmittel (4) ein Verbindungselement (4a) aufweist, welches beide Gelenkteile (5,8) und das gegebenenfalls vorgesehene Zwischenelement (11) durchgreift und an dem jeweiligen Gelenkteil (6,9) in axialer Richtung gegen lösende Verschiebung gesichert gehaltert ist.

10. Implantat nach einem der Ansprüche 1 bis 9, **dadurch gekennzeichnet, dass** das Gelenkteil (6,9) des oberen und unteren Implantatteils (2,3) als Gleitplatte ausgebildet ist, welche bei einer Gelenkbewegung des montierten Implantats (1) auf dem Zwischenelement (11) gleitend relativ zu diesem bewegbar ist.

11. Implantat nach einem der Ansprüche 1 bis 10, **dadurch gekennzeichnet,**
(i) **dass** an dem Gelenkteil (6,9) und/oder dem Anlageteil (5,8) eines Implantatteils im Bereich der Verdrehsicherungsmittel (41) eine außenseitig zugängliche Eingriffsöffnung (51) für einen Stellschlüssel vorgesehen ist, so dass mittels des Stellschlüssels das Verdrehsicherungsmittel in seine Sicherungsstellung überführbar ist,
und/oder
(ii) **dass** eines oder beide Gelenkteile (6,9) des Implantats (1) seitliche Angriffsflächen für ein Drehmomentübertragungswerkzeug aufweisen, welche zur Drehmomentübertragung an das Werkzeug angepasst sind, um das Gelenkteil (6,9) um seine Längsachse mittels des Werkzeuges zu verdrehen.

12. Implantat nach einem der Ansprüche 1 bis 11, **dadurch gekennzeichnet,**
(i) **dass** im montierten Zustand des Implantats (1) das obere und untere Implantatteil (2,3) gegeneinander und/oder gegen das Zwischenelement (11) um die Implantatlängsachse (1a) verdrehbar sind,
und/oder
(ii) **dass** im montierten Zustand des Implantats (1) das obere und untere Implantatteil (2,3) gegeneinander und/oder gegen das Zwischenelement (11) in einer Richtung quer zur Implantatlängsachse (1a) verschiebbar sind.

13. Implantat nach einem der Ansprüche 1 bis 12, **dadurch gekennzeichnet, dass** das Anlageteil (5,8) eine dem zugeordneten Knochen zugewandte durchgehend geschlossene Oberfläche aufweist.

## Claims

1. Joint implant comprising an upper and a lower implant part (2, 3) articulated to each other to form a joint, and comprising an implant longitudinal axis (1 a), each of the upper and lower implant parts (2, 3) having a joint part (6, 9) comprising the joint area and an abutment part (5, 8) that can be abutted on and supported against respectively facing bone, the joint part (6, 9) and the abutment part (5, 8) of each respective implant part (2, 3) being detachably connected to each other by respective fixing devices, said two joint parts (6, 9) of the implant parts (2, 3) each having a joint area (7, 10) and said two joint areas (7, 10) cooperating with an intermediate element (11) having upper and lower sides (12, 13) as contact surfaces, thus forming two sub-joints, and a connecting device (4) being provided interconnecting the upper and lower implant parts (2, 3) to form a coherent assembly, wherein the connecting device (4) can change position relative to the upper and lower joint parts (6, 9), **characterized in that** said two implant parts (2, 3) can freely twist against each other and against the intermediate element (11), that the fixing devices of the abutment and joint parts (5, 8; 6, 9) are constructed as form-closure devices (35, 36; 37) fixable or fixed to one another by twisting the joint part (6, 9) and the abutment part (5, 8) of the respective implant part (2, 3) relative to one another, that the cooperating form-closure devices (35, 36; 37) are secured against twisting relative to one another in a twisted position by anti-twist safeguarding devices, and that said anti-twist safeguarding devices comprise locking tongues (41).

2. Implant according to claim 1, **characterized in that** the form-closure devices (35, 36; 37) can be joined to one another in a longitudinal direction of the implant by being moved to a joining position and can be positively fixed to one another in the joining position by being twisted against each other with reference to the joining direction.

3. Implant according to claims 1 or 2, **characterized in that** the fixing device each includes a male and a female form-closure device (35, 36; 37) that cooperate for fixing, and that the male form-closure device comprises a pin with radially protruding holding areas (36) and the female form-closure device (37) comprises a non-round seat including at least one undercut and that the form-closure devices (35, 36; 37) can be joined to one another in a joining position and can be moved to a fixing position by being twisted relative to each other.

4. Implant according to one of the claims 1 to 3, **characterized in that** said anti-twist safeguarding devices include locking devices (41, 42) or are form-closure devices that can change position by means of an adjustment wrench.

5. Implant according to claim 4, **characterized in that** two anti-twist safeguarding devices (41, 42) are provided on one implant part (2, 3) which secure the joint part (6, 9) and the abutment part (5, 8) against twisting to one another in opposite twisting directions.

6. Implant according to one of the claims 1 to 5, **characterized in that** the fixing devices formed as form-closure devices (35, 36; 37) have a respective longitudinal axis and that portions with a larger or smaller distance from the longitudinal axis are provided on the joint and/or abutment part (5, 8; 6, 9) and that said anti-twist safeguarding devices (41, 42) are arranged on a portion with a larger radial distance from the longitudinal axis.

7. Implant according to one of the claims 1 to 6, **characterized in that** the fixing area of the abutment part (5, 8) includes a seat for the joint part (6, 9) and the joint part can be inserted into said seat from the surface facing the abutment part in the mounted state of the implant.

8. Implant according to one of the claims 1 to 7, **characterized in that** said abutment part or joint part (5, 8; 6, 9) includes through-holes (51) on a continuous lateral surface thereof which communicate with the seat for the respective other one of the said two parts.

9. Implant according to one of the claims 1 to 8, **characterized in that** the connecting device (4) inlcudes a connecting element (4a) that penetrates both joint parts (5, 8) and the intermediate element (11), if provided, and is supported against the respective joint part (6, 9) so as to be secured against a displacement in an axial direction which would cause the parts to separate.

10. Implant according to one of the claims 1 to 9, **characterized in that** the joint part (6, 9) of the upper and lower implant parts (2, 3) is formed as a sliding plate that can slide on, and relative to, the intermediate element (11) during an articulated motion of the mounted implant (1).

11. Implant according to one of the claims 1 to 10, **characterized in that**
(i) that an externally accessible engagement opening (51) for an adjustment wrench is provided on the joint part (6, 9) and/or on the abutment part (5, 8) of an implant part in the region of the anti-twist safeguarding device so that the anti-twist safeguarding device can be moved to its safeguarding position by means of the adjustment wrench and/or
(ii) that one or both joint parts (6, 9) of the implant (1) define lateral engagement surfaces for a torque transmitting tool which are adapted to the tool, for torque transmission in order to turn the joint part (6, 9) about its longitudinal axis by means of said tool.

12. Implant according to one of the claims 1 to 11, **characterized in that**
(i) in the mounted state of the implant (1), the upper and lower implant parts (2, 3) can be twisted about the longitudinal axis (1 a) of the implant relative to each other and/or relative to the intermediate element (11), and/or
(ii) that in the mounted state of the implant (1), the upper and lower implant parts (2, 3) can be displaced relative to each other and/or relative to the intermediate element (11), in a direction transverse to the longitudinal axis (1a) of the implant.

13. Implant according to one of the claims 1 to 12, **characterized in that** the abutment part (5, 8) has a continuous closed surface toward associated bone when implanted.

## Revendications

1. Implant pour articulation, comprenant un élément supérieur et inférieur d'implant (2, 3) articulés l'un à l'autre en forment une articulation, et un axe longitudinal d'implant (1a), les éléments supérieur et inférieur d'implant (2, 3) respectivement présentant un élément d'articulation (6, 9) entourant la zone d'articulation et un élément d'appui (5, 8) pouvant être appliqué et supporté contre l'os respectivement faisant face, ledit élément d'articulation (6, 9) et ledit élément d'appui (5, 8) de l'élément d'implant (2, 3) respectif étant reliés entre eux de façon amovible, les deux éléments d'articulation (6, 9) des éléments d'implant (2, 3) présentant chacun une zone d'articulation (7, 10), les deux zones d'articulation (7,10) coopérant avec un élément intermédiaire (11) comportant des faces supérieure et inférieure opposées (12, 13) en tant que surfaces d'appui en forment deux articulations partielles, et un moyen de connexion (4) étant prévu et connectant les éléments d'implant supérieur et inférieur (2, 3) en formant un ensemble cohérant, le moyen de connexion (4) étant réglable en position par rapport aux éléments d'articulation supérieur et inférieur, **caractérisé en ce que** les deux éléments d'implant peuvent librement tourner l'un par rapport à l'autre et par rapport à l'élément intermédiaire (11), que les moyens de fixation des éléments d'appui et d'articulation (5, 8; 6, 9) sont réalisés comme moyens d'assemblage par complémentarité de formes (35, 35; 37) qui peuvent être ou sont fixés les uns aux autres sous torsion de l'élément d'articulation (6, 9) et de l'élément d'appui (5, 8) de l'élément d'implant (2, 3) respectif, que les moyens d'assemblage par complémentarité de formes (35, 35; 37), dans une position prise par rotation, sont bloqués en rotation l'un par rapport à l'autre par des moyens de sécurité anti-rotation (41, 42), et que les moyens de sécurité anti-rotation comportent des languettes d'encliquetage (41).

2. Implant selon la revendication 1, **caractérisé en ce que** les moyens d'assemblage par complémentarité de formes (35, 36; 37) sont réalisés de sorte qu'ils peuvent être assemblés en les mettant dans une position d'assemblage dans la direction longitudinale de l'implant et qu'ils peuvent être fixés les uns par rapport aux autres dans la position d'assemblage par rotation les uns contre les autres dans la direction d'assemblage en formant une complémentarité de formes.

3. Implant selon la revendication 1 ou 2, **caractérisé en ce que** le moyen de fixation respectivement comporte un moyen d'assemblage par complémentarité de formes male et femelle (35, 36; 37) coopérant pour la fixation, et que le moyen d'assemblage par complémentarité de formes male présente un tenon (35) avec des zones de retenue (36) saillant radialement, et le moyen d'assemblage par complémentarité de formes femelle (37) présente un logement non circulaire avec au moins une contre-dépouille, et que les moyens d'assemblage par complémentarité de formes (35, 36; 37), dans une position d'assemblage, peuvent être assemblés et transférés dans une position de fixation par rotation les uns par rapport aux autres.

4. Implant selon l'une des revendications 1 à 3, **caractérisé en ce que** les moyens de sécurité anti-rotation comportent des moyens d'encliquetage (41, 42) ou sont des moyens d'assemblage par complémentarité de formes réglables en position au moyen d'une clé de réglage.

5. Implant selon la revendication 4, **caractérisé en ce que** sur un élément d'implant (2, 3) sont prévus deux moyens de sécurité anti-rotation (41, 42) fixant l'élément d'articulation (6, 9) et l'élément d'appui (5, 8) contre la rotation l'un par rapport à l'autre dans des directions de rotation opposées.

6. Implant selon l'une des revendications 1 à 5, **caractérisé en ce que** les moyens de fixation réalisés comme moyens d'assemblage par complémentarité de formes respectivement présentent un axe longitudinal et que sur l'élément d'articulation et/ou sur l'élément d'appui sont prévues des parties à distance radiale plus grande et plus petit de l'axe longitudinal et que les moyens de sécurité anti-rotation (41, 42) sont arrangés sur les parties à distance radiale plus grande de l'axe longitudinal.

7. Implant selon l'une des revendications 1 à 6, **caractérisé en ce que** la partie de fixation de l'élément d'appui (5, 8) comporte un logement pour l'élément d'articulation (6, 9), dans ce logement peut être inséré l'élément d'articulation depuis la surface tournée vers l'élément d'appui lorsque l'implant est monté.

8. Implant selon l'une des revendications 1 à 7, **caractérisé en ce que** ledit élément d'appui ou d'articulation (5, 8; 6, 9) présente des ouvertures de passage (51) sur sa face latérale périphérique qui sont en communication avec le logement pour l'autre desdits éléments.

9. implant selon l'une des revendications 1 à 8, **caractérisé en ce que** le moyen de connexion (4) comporte un élément de connexion (4a) traversant les deux éléments d'articulation (5, 8) et l'élément intermédiaire prévu le cas échéant, et étant sécurisé sur l'élément d'articulation (6, 9) respectif contre un déplacement dégageant dans la direction axiale.

10. Implant selon l'une des revendications 1 à 9, **caractérisé en ce que** l'élément d'articulation (6, 9) des éléments d'implant supérieur et inférieur (2, 3) est réalisé comme plaque glissante mobile de manière glissante sur et relativement à l'élément intermédiaire (11) lors d'un mouvement articulé de l'implant (1) monté.

11. implant selon l'une des revendications 1 à 10, **caractérisé en ce que**
(i) sur l'élément d'articulation (6, 9) et/ou sur l'élément d'appui (5, 8) d'un élément d'implant est prévu, dans la zone des moyes de sécurité anti-rotation (41), une ouverture d'engagement (51) accessible de l'extérieur pour une clé de réglage de sorte que ledit moyen de sécurité anti-rotation peut être placé dans la position de sécurité au moyen de ladite clé de réglage, et/ou
(ii) l'un ou les deux éléments d'articulation (6, 9) de l'implant (1) présentent des surfaces d'engagement latérales pour un outil de transmission de couple, lesdites surfaces d'engagement, pour la transmission du couple, étant adaptées audit outil pour tourner l'élément d'articulation (6, 9) autour son axe longitudinal par ledit outil.

12. Implant selon l'une des revendications 1 à 11, **caractérisé en ce que**
(i) lorsque l'implant (1) est monté, les éléments supérieur et inférieur d'implant (2, 3) peuvent être tournés l'un contre l'autre ou contre l'élément intermédiaire (11) autour l'axe longitudinal (1a), et/ou
(ii) lorsque l'implant (1) est monté, les éléments supérieur et inférieur d'implant (2, 3) peuvent être déplacés l'un contre l'autre ou contre l'élément intermédiaire (11) dans une direction transversale par rapport à l'axe longitudinal (1a) de l'implant.

13. Implant selon l'une des revendications 1 à 12, **caractérisé en ce que** l'élément d'appui (5, 8) présente une surface continuellement fermée et tournée vers l'os associé.
